# EUROPÄISCHE PATENTSCHRIFT

(11) **EP 3 111 964 B1**
(45) Veröffentlichungstag und Bekanntmachung des Hinweises auf die Patenterteilung: **13.06.2018**
(21) Anmeldenummer: 16175814.9
(22) Anmeldetag: 22.06.2016
(51) Int. Cl.: A61L 2/26

(54) **VERSCHLUSSMECHANISMUS UND SIEBKORB MIT EINEM DECKEL, DER DEN VERSCHLUSSMECHANISMUS UMFASST**
LOCKING MECHANISM AND SCREEN BASKET WITH A LID, COMPRISING THE LOCKING MECHANISM
MECANISME DE VERROUILLAGE ET CREPINE DOTEE D'UN COUVERCLE INCLUANT LE MECANISME DE VERROUILLAGE

(30) Priorität: 03.07.2015 DE 202015103523 U
(43) Veröffentlichungstag der Anmeldung: 04.01.2017
(73) Patentinhaber: Ermis Medizintechnik e.K., 78532 Tuttlingen (DE)
(72) Erfinder: Ermis, Mehmet, 78532 Tuttlingen (DE); Kurz, Thomas, 78532 Tuttlingen (DE)
(74) Vertreter: Straub, Bernd

(56) Entgegenhaltungen:
- EP-A2- 1 431 481
- EP-A2- 2 261 445
- WO-A1-2015/062924
- WO-A1-2016/083595
- DE-A1-102012 111 096
- DE-U1- 9 203 630
- DE-U1- 29 504 063
- DE-U1-202007 003 395
- DE-U1-202010 016 040

## Beschreibung

### TECHNISCHES GEBIET

Die Erfindung betrifft einen Verschlussmechanismus und einen Siebkorb mit einem Deckel, der den Verschlussmechanismus umfasst.

### STAND DER TECHNIK

Aus dem Stand der Technik sind unterschiedliche Verschlussmechanismen bekannt. Beispielsweise für Siebkörbe, wie sie zum Beispiel für Sterilisierbehälter in der Medizintechnik verwendet werden, sind zur Befestigung eines Deckels an dem Siebkorbbehälter beispielsweise Bügelverschlüsse, Verschlüsse mit Drehmechanismen, Schieberiegelverschlüsse oder Schnappverschlüsse bekannt.

Beispielsweise zeigt die DE 20 2010 016 040 U1 eine Verriegelungsvorrichtung einer Siebschale zum Aufnehmen von zu sterilisierendem Gut, die über ein am Deckel angebrachtes Arretierelement hinweg, unter Ausnutzung der Federeigenschaften der Verriegelungsvorrichtung und des Arretierelementes, in eine den Deckel verriegelnde Position verschwenkbar ist. Hierbei ist das Arretierelement ein vom Deckel abstehender Zapfen, der orthogonal von der Oberfläche des Deckels absteht, so dass der Deckel, wenn er angehoben wird, mit dem Aufnahmebehälter verbunden bleibt.

Siebkörbe selbst sind beispielsweise in den Patentanmeldungen DE 10 2012 016 970 A1, der DE 10 2011 052 001 A1 oder dem Gebrauchsmuster DE 20 2010 005 788 U1 beschrieben.

Weiterhin ist aus dem Deutschen Gebrauchsmuster DE 295 04 063 U1 ein Drahtkorb mit Seitenwänden aus Drahtmaschen mit einem Spanndeckel bekannt, der im Inneren des Drahtkorbes mittels an dem Spanndeckel angebrachten Raststiften befestigbar ist. Dabei sind die Raststifte gegen die Seitenwände federnd vorgespannt und greifen in an den Seitenwänden vertikal verlaufende Rastschienen mit Rastkerben ein und verschießen damit den Drahtkorb. Durch horizontales Verschieben der Raststifte mit dem verbundenen Handgriff gegen die Spannfedern kann der Verschluss geöffnet werden und der Spanndeckel aus dem Innern des Drahtkorbes entnommen werden. Dieser Verschluss und der Drahtkorb erweisen sich in ihrer Handhabung als sehr umständlich.

### BESCHREIBUNG DER ERFINDUNG

Aufgabe der Erfindung ist es, einen verbesserten Verschlussmechanismus und einen Siebkorb mit einem Deckel, der diesen Verschlussmechanismus umfasst, bereitzustellen. Die Verbesserung soll bei verbesserter Handhabung eine große Sicherheit gegen unerwünschtes Öffnen gewährleisten.

Diese Aufgabe wird durch einen verbesserten Verschlussmechanismus gemäß Anspruch 1 und einen Siebkorb gemäß Anspruch 10 beziehungsweise 11 gelöst.

Vorteilhafte Ausgestaltungen der Erfindung sind Gegenstand der Unteransprüche.

Ein erfindungsgemäßer Verschlussmechanismus umfasst eine Hebelasche, einen Schieber und einen federnd gelagerten Gegenschieber, wobei die Hebelasche kippbar um eine erste Achse gelagert ist und mit einem Schiebevorsprung verbunden ist, der so ausgebildet ist, dass er für einen Öffnungsvorgang beim Anheben der Hebelasche den Schieber senkrecht zur ersten Achse verschiebt und wobei der Gegenschieber durch Federkraft entgegen der Verschieberichtung des Schiebers gedrückt wird. Durch Anheben der Hebelasche beim Öffnungsvorgang des Verschlussmechanismus dreht sich die Hebelasche um die erste Achse und ein Schiebevorsprung der Hebelasche wird durch die Kippbewegung gegen einen Schieber gedrückt, so dass der Schieber eine Translationsbewegung senkrecht zur ersten Achse ausführt und dadurch beispielsweise die Verriegelung eines Deckels, einer Tür, einer Schublade oder ähnlichem freigibt. Hierdurch gelingt es, eine gute Handhabung des erfindungsgemäßen Verschlussmechanismus zu erreichen.

Zusätzlich zu der Translationsbewegung des Schiebers wird ein federnd vorgespannter Gegenschieber entgegen der Verschieberichtung des Schiebers gedrückt. Durch eine spezielle Formgebung des Gegenschiebers, beispielsweise als schräge Rampe geformt, kann die Translationsbewegung des Gegenschiebers in Zusammenwirkung mit einem Anschlag, der beispielsweise durch einen Verschlusseingriff vorzugsweise in Form eines nach innen gebogenen Randes eines Behälters oder eines an der Innenwand eines Behälters angeordneten Vorsprungs gebildet werden kann eine Kraftumlenkung bewirken, so dass beispielsweise ein Deckel eines Behälters durch die Federkraft des Gegenschiebers angehoben wird. Dieses Anheben bewirkt eine besonders sichere Trennung des Verschlussmechanismusses vom Gegenstück beispielsweise des Deckels mit Verschlussmechanismus vom Behälter, so dass eine besonders vorteilhafte, erleichterte Handhabung gewährleistet ist.

Gemäß einer bevorzugten Ausführungsform ist die erste Achse, die ein Drehlager für die Hebelasche und den Schiebevorsprung bildet, einstückig durch Vorsprünge an der Hebelasche ausgebildet. Durch die einstückige Ausbildung gelingt es, die Anfälligkeit der Hebelasche zum Betätigen des Verschlussmechanismusses zu reduzieren und dadurch die Sicherheit bei guter Handhabung der Hebelasche zu gewährleisten.

Gemäß einer weiteren vorteilhaften Ausführungsform ist der Schiebevorsprung einstückig an der Hebelasche ausgebildet. Um durch die Kippbewegung der Hebelasche eine Kraft im Wesentlichen parallel zur Montageoberfläche des Verschlussmechanismusses zu erzeugen, ist der Schiebevorsprung senkrecht zur Oberfläche der Hebelasche ausgebildet.

Gemäß einer weiteren vorteilhaften Ausführungsform weist der Schieber oder ein am Schieber angeordnetes und fest mit diesem verbundenes Anschlagselement eine Aussparung insbesondere eine lochförmige Aussparung zur Aufnahme des Gegenschiebers und damit zur wechselseitigen Führung des Schiebers respektive des Gegenschiebers auf. Dabei ist das Anschlagselement vorzugsweise so ausgebildet, dass es sich quer zur Verschieberichtung des Schiebers und in Richtung des Schiebevorsprungs erstreckt und damit geeignet ist, eine Bewegung des Schiebevorsprungs zu übernehmen und auf den Schieber so zu übertragen, dass eine Verschiebung in Verschieberichtung oder entgegen dieser bewirkt wird. Die Ausnehmung kann dabei durch Entfernen von Teilen des Schiebers oder des Anschlagselementes gebildet sein oder auch durch Hinzufügen von Vorsprüngen, die eine Ausnehmung umschließen, gebildet werden. Durch das Vorsehen der Führung für den Gegenschieber beziehungsweise das Vorsehen einer wechselseitigen Führung des Schiebers beziehungsweise des Gegenschiebers ist ein sehr sicherer Verschlussmechanismus geschaffen, der sich durch einen sehr verlässlichen Betrieb auch unter schwierigen äußeren Umständen auszeichnet.

Gemäß einer bevorzugten Ausführungsform der Erfindung ist der Schieber oder der Gegenschieber flächig ausgebildet und bildet ein Gehäuse, das den restlichen Verschlussmechanismus ganz oder teilweise umschließt. Dadurch gelingt es, den erfindungsgemäßen Verschlussmechanismus vor Beschädigung zu schützen und dauerhaft funktionsfähig zu halten. Vorzugsweise wird dabei in den flächigen Schieber eine Ausnehmung eingebracht, die eine wechselseitige Führung des Schiebers respektive des Gegenschiebers ermöglicht und dadurch die Funktionalität des Schiebers bzw. des Gegenschiebers im Besonderen gewährleistet. Auch hat es sich als vorteilhaft erwiesen, den flächigen Schieber beziehungsweise Gegenschieber mit Hilfe einer Führung in Verschieberichtung oder entgegen der Verschieberichtung zu führen und dadurch ein sicheres geradliniges Gleiten zu ermöglichen.

Gemäß einer weiteren vorteilhaften Ausführungsform weist der erfindungsgemäße Verschlussmechanismus außerdem wenigstens ein Federelement auf, das mit dem Gegenschieber zusammenwirkt und geeignet ist, eine Federkraft entgegen der Verschieberichtung des Schiebers zu erzeugen. Dieses Federelement kann beispielsweise eine Spiralfeder oder aber auch eine Blattfeder sein. Damit gelingt es, den Gegenschieber entgegen der Verschieberichtung des Schiebers vorzuspannen und diesen dazu zu verwenden, dass eine sichere Fixierung mit Hilfe des Zusammenwirkens des Gegenschiebers und des Schiebers erreicht wird.

Gemäß einer bevorzugten Ausführungsform des Verschlussmechanismusses ist wenigstens ein Federelement vorgesehen, das mit dem Schieber zusammenwirkt und geeignet ist, eine Federkraft entgegen der Verschieberichtung des Schiebers zu erzeugen und damit den Schieber federbewehrt hinter einen Vorsprung oder in eine Vertiefung einzubringen und dadurch einen sicheren Verschluss zu erreichen.

Als besonders vorteilhaft hat es sich erwiesen, am Schieber und/oder am Gegenschieber einen schräg zur Verschieberichtung verlaufenden Abschnitt vorzusehen. Durch diesen schräg verlaufenden Abschnitt gelingt es, die Handhabung des Verschlussmechanismusses zu verbessern und zusätzlich die Verschlusssicherheit zu erhöhen.

Durch das Vorsehen des schrägen Abschnittes im Bereich des Endes des Schiebers wird es möglich, dass sich der Schieber, sobald er mit dem schrägen Abschnitt auf einen Vorsprung aufläuft, in Verschieberichtung insbesondere entgegen der Federkraft des mit dem Schieber verbundenen Federelementes bewegt, bis der Schieber wieder entgegen der Verschieberichtung in den freien Raum hinter dem Vorsprung oder in den Raum einer Ausnehmung hineingleitet und dadurch ein sicheres Verschließen gewährleistet.

Durch das Vorsehen des schrägen Abschnittes und des Federelementes am Schieber ist die Funktionalität des Verschlussmechanismusses auch bei wenig präziser Positionierung des Verschlussmechanismusses gegenüber dem zu verschließenden Gegenstand gewährleistet.

Durch das Vorsehen eines entsprechenden schrägen Abschnittes am Gegenschieber gelingt es, die Verschiebebewegung des Gegenschiebers beim Auftreffen auf einen Anlaufpunkt in eine nach oben gerichtete Bewegung umzulenken und dadurch das Abheben des Gegenstandes, an dem der Verschlussmechanismus befestigt ist, von dem Gegenstück zu bewirken und dadurch die Handhabung wesentlich zu vereinfachen, ohne dass die Sicherheit des Verschlussmechanismusses beeinträchtigt ist. Werden der Schieber und der Gegenschieber mit einem schrägen Abschnitt versehen, hat es sich als vorteilhaft erwiesen, diese Abschnitte gleich oder etwa gleich schräg zur Verschieberichtung auszubilden und dadurch eine entsprechende Handhabung zu erreichen. Auch ist es dadurch möglich, dass die schrägen Abschnitte eine gemeinsame Fläche bildend zum Liegen kommen und dadurch die Gefahr eines ungewollten Einhakens und Blockierens des Verschlusses verringert wird.

Dabei ist der Gegenschieber vorzugsweise so ausgebildet, dass sich an dem schrägen Abschnitt ein gerader Endabschnitt des Gegenschiebers anschließt, der im Wesentlichen die gleiche Ausrichtung wie der restliche Schieber und damit im Wesentlichen eine Ausrichtung wie die Verschieberichtung zeigt. Durch diesen geraden Abschnitt wird es möglich, einen Vorsprung, der beispielsweise ein nach innen gebogener Rand eines Behälters sein kann, von den Enden des Schiebers beziehungsweise des Gegenschiebers zu umschließen und dadurch eine sichere Positionierung des Verschlussmechanismusses relativ zum zu verschließenden Gegenstand zu schaffen. Der Vorsprung wird bevorzugt durch den Verschlusseingriff gebildet, hinter den der Schieber des Verschlussmechanismusses zum Schließen eingreift. Der Vorsprung wird dabei bevorzugt im Bereich des oberen Randes an der Innenseite eines mit einem Deckel zu verschließenden Behälters angeordnet. Alternativ kann er auch beabstandet zum oberen Rand des Behälters beispielsweis in der Mitte an der Innenseite der Seitenwand des Behälters angeordnet sein. Hierdurch ist eine erhöhte Sicherheit des Verschlussmechanismusses erreicht.

Gemäß einer weiteren vorteilhaften Ausführungsform weist der erfindungsgemäße Verschlussmechanismus einen weiteren Schiebevorsprung auf, der mit der Hebelasche verbunden ist und der gemeinsam mit dem anderen Schiebevorsprung ein am Schieber angeordnetes Anschlagselement umfasst. Somit ist diese Ausführung geeignet, eine bidirektionale Bewegung also ein Vor- und Zurückschwenken des Schiebevorsprungs in die Verschieberichtung und entgegen der Verschieberichtung auf das Anschlagselement zu übertragen und dadurch eine bidirektionale, translatorische Bewegung des Schiebers in Verschieberichtung und entgegen der Verschieberichtung zu erzeugen und damit ein sicheres Öffnen und Verschließen des Verschlussmechanismusses zu gewährleisten.

Alternativ oder ergänzend hat es sich auch bewährt, wenigstens einen Schiebevorsprung mit dem Schieber mechanisch so zu verbinden, dass eine bidirektionale Bewegung des Schiebevorsprungs in eine bidirektionale Bewegung des Schiebers übertragen wird. Dies wird beispielsweise durch ein Verbindungselement beispielsweise in Form einer Kette, eines Übertragungsschenkels, eines Gummibandes oder eines Seils, welches einerseits am Schiebevorsprung und andererseits am Schieber befestigt ist und damit eine Kraft von einem auf das andere Element übertragen kann, erreicht.

Darüber hinaus hat es sich als vorteilhaft erwiesen, eine Hebelasche nicht nur einem Schieber und einem einzigen Gegenschieber zuzuordnen und diese mit Hilfe des Schiebevorsprungs zu bewegen, sondern einer Hebelasche mit Schiebevorsprung mehrere Schieber und/oder mehrere zugeordnete Gegenschieber zuzuordnen. Diese bewirken gemeinsam beim Öffnungsvorgang durch das Anheben der Hebelasche eine Verschiebung der Schieber senkrecht zur ersten Achse der Hebelasche und entsprechend ein Drücken der Gegenschieber durch eine Federkraft entgegen der Verschieberichtung des Schiebers. Durch dieses mehrfache Vorsehen der Schieber beziehungsweise Gegenschieber bei einer einzigen Hebelasche ist einerseits eine einfache Handhabung bei vergrößerter Verschlusssicherheit gewährleistet.

Ein erfindungsgemäßer Siebkorb umfasst einen Behälter zur Aufnahme von beispielsweise Sterilgut, wie es in der Medizintechnik verwendet wird, beispielsweise Operationsbesteck oder andere chirurgische Instrumente. Der Behälter umfasst mindestens einen Verschlusseingriff, Sieböffnungen und mindestens eine Deckelbefestigungsöffnung. Dabei ist ein Verschlusseingriff vorzugsweise als ein nach innen gebogener, oberer Rand oder ein an der Innerseite des Behälters angeordneter Vorsprung ausgebildet. Der erfindungsgemäße Siebkorb enthält außerdem einen Deckel, der über mindestens einen Befestigungsvorsprung mit einer der Deckelbefestigungsöffnungen des Behälters in Eingriff bringbar ist und weist einen oder mehrere der oben beschriebenen erfindungsgemäßen Verschlussmechanismen auf. Im geschlossenen Zustand des Siebkorbes greift der Schieber des Verschlussmechanismusses unter den beispielsweise als nach innen gebogener oberer Rand oder als nach innen gerichteter Vorsprung ausgebildeten Verschlusseingriff des Behälters ein und verhindert damit zusammen mit dem in Eingriff gebrachten Befestigungsvorsprung ein unbeabsichtigtes Öffnen des Deckels. Beim Öffnen des Deckels durch Anheben der Hebelasche des Verschlussmechanismusses verschiebt sich der Schieber senkrecht zur ersten Achse und löst dadurch den Eingriff unter dem oberen Rand oder Vorsprung bzw. Verschlusseingriff des Behälters. Hierdurch wird der Deckel für das Abheben und damit Trennen des Deckels vom Behälter freigegeben. Der Behälter kann nun befüllt oder die Füllung aus dem Behälter entnommen werden. Dadurch ist die Handhabung wesentlich erleichtert.

Ein alternativer erfindungsgemäßer Siebkorb umfasst einen mit mindestens einem Verschlusseingriff, der vorzugsweise einen nach innen gebogenen oberen Rand oder einen an der Innenseite des Behälters angeordneten Vorsprung darstellt, und mit Sieböffnungen versehenen Behälter sowie einen Deckel, der wenigstens einen oder mehrere der oben beschriebenen Verschlussmechanismen aufweist. Im geschlossenen Zustand des Siebkorbes greifen der oder die Schieber der Verschlussmechanismen unter den oberen Rand oder Vorsprung bzw. Verschlusseingriff des Behälters ein und verhindern damit ein unbeabsichtigtes Öffnen des Deckels. Beim Öffnen des Deckels durch Anheben der Hebelasche der Verschlussmechanismen verschiebt sich der Schieber senkrecht zur ersten Achse, wodurch sich der Eingriff unter dem oberen Rand oder Vorsprung bzw. Verschlusseingriff des Behälters löst und der Deckel für das Abheben freigegeben wird. Bei dieser alternativen Ausbildung des Siebkorbes mit erfindungsgemäßen Verschlussmechanismen wird der Deckel an zwei
oder mehr insbesondere gegenüberliegenden Stellen durch den Eingriff unter den Rand oder Vorsprung bzw. Verschlusseingriff des Behälters gesichert und damit im geschlossenen Zustand bewahrt, bis durch Anheben der Hebelasche die Schieber verschoben werden und der Deckel freigegeben wird. Die beiden alternativen, erfindungsgemäßen Siebkörbe zeichnen sich durch eine einfache Handhabung des Verschlussmechanismusses zum Öffnen beziehungsweise Schließen des Siebkorbes aus.

Gemäß einer weiteren vorteilhaften Ausführungsform wird der Gegenschieber durch Federkraft entgegen der Verschieberichtung des Schiebers zum oberen Rand oder Vorsprung bzw. Verschlusseingriff des Behälters hingedrückt und erzeugt dort eine nach oben gerichtete Kraft. Dies erfolgt bevorzugt durch eine besondere Form des Gegenschiebers, beispielsweise durch das Vorsehen eines schrägen Abschnittes im Bereich des vorderen Endes des Gegenschiebers. Hierdurch kann die Translationsbewegung des Gegenschiebers eine nach oben gerichtete Kraft erzeugen, die dazu führt, dass beispielsweise der Deckel des Siebkorbes von dem Behälter abgehoben wird. Alternativ dazu hat es sich auch bewährt, den Rand oder Vorsprung bzw. den Verschlusseingriff des Behälters mit einer schräg zur Verschieberichtung verlaufenden Anlauffläche zu versehen, auf die der Gegenschieber insbesondere durch die Federkraft des vorgesehenen Federelementes aufgedrückt wird und dadurch zu einem Anheben des Deckels mit dem Verschlussmechanismus führt. Hierdurch gelingt es, die Handhabung des Siebkorbes mit dem erfindungsgemäßen Verschlussmechanismus in besonderem Maß zu verbessern.

Vorzugsweise werden zur Erreichung einer besonders guten Handhabung und eines sicheren Verschließens des Deckels des Siebkorbes mehrere erfindungsgemäße Verschlussmechanismen am Deckel vorgesehen, die an verschiedenen Seiten des Deckels verteilt angeordnet sind. Dabei hat es sich besonders bewährt, Verschlussmechanismen an gegenüberliegenden Seiten des Deckels insbesondere paarweise vorzusehen. Dadurch ist ein sicheres Verschließen bei erhaltener, vorteilhafter, einfacher Handhabung gewährleistet.

Gemäß einer bevorzugten Ausführungsform des Siebkorbes ist die Hebelasche des Verschlussmechanismus in einer Vertiefung der Oberseite des Deckels versenkt angeordnet und die erste Achse ist in seitlichen Aussparungen der Vertiefung gelagert. Die erste Achse und die seitlichen Aussparungen bilden dabei ein Drehlager für die Verschwenkung der Hebelasche mit dem Schiebevorsprung. Durch diese vorteilhafte Ausführungsform der seitlichen Aussparungen in der Vertiefung kann auf separate Lagerelemente verzichtet werden. Dadurch gelingt es, die Reparaturanfälligkeit des Siebkorbes zu verbessern und dadurch einen sehr sicheren Verschlussmechanismus für den erfindungsgemäßen Siebkorb zu schaffen.

Gemäß einer weiteren vorteilhaften Ausführungsform eines erfindungsgemäßen Siebkorbes weist der Deckel mindestens zwei Befestigungsvorsprünge auf der gleichen Seite und gegenüber des Verschlussmechanismusses auf und der Behälter umfasst mindestens zwei insbesondere vier Deckelbefestigungsöffnungen, wobei sich immer zwei der Deckelbefestigungsöffnungen paarweise gegenüberliegen. Durch diese vorteilhafte symmetrische Ausführungsform ist es möglich, den Deckel in zwei verschiedenen Richtungen, jeweils 180° gedreht, an dem Behälter anzubringen. Diese Ausführungsform des erfindungsgemäßen Siebkorbes zeichnet sich durch eine besonders vorteilhafte Handhabung aus.

Gemäß einer weiteren vorteilhaften Ausführungsform umfasst ein quaderförmiger Siebkorb je eine Stapelmulde in jeder der vier Ecken des Deckels und je einen Stapelfuß in jeder der vier Ecken des Bodens des Behälters. Durch diese vorteilhafte Ausführungsform kann ein Verrutschen gestapelter Siebkörbe verhindert werden. Gerade durch das Vorsehen von Stapelfüßen beziehungsweise Stapelmulden, die einen erfindungsgemäßen Verschlussmechanismus umschließen, gelingt es, den Bereich des Deckels, in dem der Verschlussmechanismus angeordnet ist, besonders vor Verformung zu schützen und dadurch einen besonders verlässlichen und sicheren Verschlussmechanismus des erfindungsgemäßen Siebkorbes sicherzustellen. Hierbei wirken Stapelmulden beziehungsweise Stapelfüße versteifend auf den Deckelbereich, in dem der erfindungsgemäße Verschlussmechanismus angeordnet ist.

Gemäß einer weiteren vorteilhaften Ausführungsform enthält der Behälter zwei ausziehbare Griffbügel und der Deckel weist zwei Griffaussparungen auf, die derart angeordnet sind, dass die Griffbügel des Behälters bei geschlossenem Deckel durch die Griffaussparungen des Deckels durchführbar sind. Diese vorteilhafte Ausführungsform erlaubt es, die Griffbügel des Behälters platzsparend im Inneren des Behälters anzuordnen, so dass es auf der einen Seite trotzdem möglich ist, den Behälter an den zwei ausziehbaren Griffbügeln sicher zu tragen, es aber andererseits trotzdem möglich ist, mehrere Siebkörbe auf Anschlag nebeneinander zu lagern, ohne dass diese sich an außen angeordneten Griffbügeln verhaken können. Zusätzlich gelingt es durch diese Ausbildung, den Deckel mit dem erfindungsgemäßen Verschlussmechanismus im Gewicht zu reduzieren und dadurch eine bessere Handhabung des Siebkorbes beim Verschließen und Handhaben des Deckels zu gewährleisten, ohne dass die Handhabung des gesamten geschlossenen Siebkorbes mit Griffbügeln beeinträchtigt ist.

Die Erfindung wird nachfolgend anhand bevorzugter Ausführungsbeispiele unter Bezugnahme auf die Abbildungen beispielhaft erläutert. Die Erfindung ist nicht auf diese bevorzugten Ausführungsbeispiele beschränkt.
- Fig. 1a: zeigt eine schematische Seitenansicht einer Ausführungsform des erfindungsgemäßen Verschlussmechanismusses.
- Fig. 1b: zeigt eine Draufsicht auf eine bevorzugte Ausführungsform einer Hebelasche des erfindungsgemäßen Verschlussmechanismusses.
- Fig. 2: zeigt in einer schematischen Ansicht ein Beispiel einer Anordnung des erfindungsgemäßen Verschlussmechanismusses in einem Deckel eines Siebkorbes.
- Fig. 3a und 3b: zeigen eine schematische Draufsicht einer beispielhaften Ausführungsform eines Deckels (Fig. 3a) und eines Behälters (Fig. 3b) eines erfindungsgemäßen Siebkorbes.

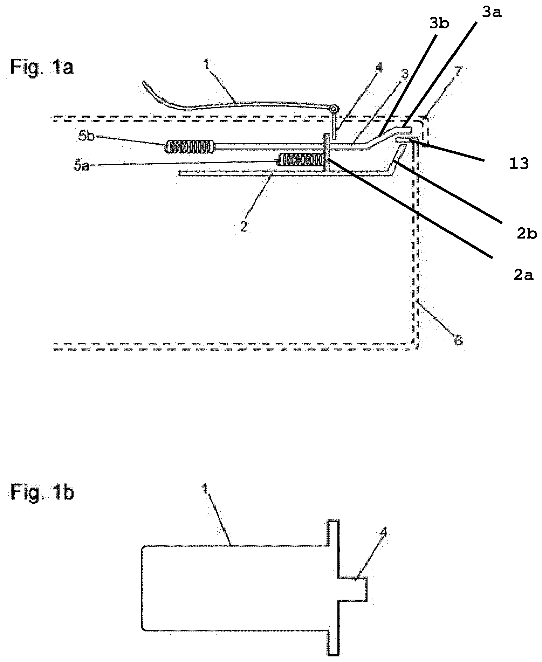

Fig. 1a zeigt schematisch eine Seitenansicht eines erfindungsgemäßen Verschlussmechanismusses. Der Verschlussmechanismus ist bevorzugt aus Metall, insbesondere aus Aluminium, Edelstahl oder einer Metalllegierung oder aber auch aus Kunststoff, wie beispielsweise Polyimid, Polyhydroxybutyrat, Polypropylen, Polytetrafluorethylen, Polyurethan oder Polyester, beziehungsweise aus einer Kombination davon gebildet. Der erfindungsgemäße Verschlussmechanismus umfasst eine Hebelasche 1, die kippbar um eine erste Achse gelagert ist und mit einen Schiebevorsprung 4 einstückig verbunden ist. Der Schiebevorsprung 4 erstreckt sich orthogonal zur Hebelasche 1 und greift durch die Deckelfläche. Beim Anheben/Kippen der Hebelasche 1 kann der Schiebevorsprung 4 an der Hebelasche 1 mit einem Schieber 2 und hier mit dem Anschlagselement 2a des Schiebers 2 zusammenwirken und eine Translationsbewegung senkrecht zur ersten Achse des Schiebers 2 erzeugen. Beim Loslassen der Hebelasche 1 kann der Schieber 2, vorteilhafterweise durch ein Federelement 5a, in die Schließposition geschoben werden und hierbei auch die Hebelasche wieder in ihre abgesenkte Position bringen.

Zum Zweck einer klaren Darstellung wurde auf die Darstellung der Mittel zur Befestigung des Verschlussmechanismusses, beispielsweise Schrauben oder Nieten, verzichtet. Durch ein Federelement 5b wird ein Gegenschieber 3 entgegen der Verschieberichtung des Schiebers 2 gedrückt. Auch hier wird auf die Darstellung des Gegenlagers für die Federelemente 5a und 5b verzichtet. Eine mögliche Ausführungsform einer solchen Lagerung der Federelemente 5a und 5b könnte beispielsweise eine Platte mit gefrästen Schlitzen zur Aufnahme der Federn und des Gegenschiebers 3 sein. Diese Platte könnte dann beispielsweise auch Gewindelöcher zur Befestigung des Verschlussmechanismusses enthalten.

Vorzugsweise kann mit Hilfe des schrägen Abschnitts 3b, der eine rampenartige Ausbildung der Vorderseite des Gegenschiebers 3 darstellt, eine Translationsbewegung des Gegenschiebers 3, verursacht durch beispielsweise ein Federelement 5b, eine vertikale Bewegung des Verschlussmechanismusses senkrecht zur Translationsbewegung des Gegenschiebers 3 bewirkt werden. Diese vertikale Bewegung führt zum Abheben des Deckels vom Behälter, die über den Verschlussmechanismus lösbar verbunden sind. Das Federelement 5b kann beispielsweise als Spiralfeder, als Blattfeder oder anderes Federelement ausgebildet sein.

An den schrägen Abschnitt 3b schließt sich am vorderen Ende des Gegenschiebers 3 ein gerader, im Wesentlichen zum restlichen Gegenschieber 3 beziehungsweise zur Verschieberichtung paralleler Abschnitt 3a an, der im geschlossenen Zustand einen oberen Anschlag für den Verschlusseingriff 13 bildet, der als ein nach innen gebogener oberer Rand 13 oder nach innen gerichteter Vorsprung ausgebildet ist und hinter den der Schieber 2 schließend eingreift und damit gemeinsam mit dem Gegenschieber 3 den Verschlusseingriff 13 bzw. Rand 13 oder Vorsprung festlegt. Dies sorgt für eine sehr sichere und gute Handhabung des erfindungsgemäßen Verschlussmechanismusses.

Wie in Fig. 1b gezeigt, kann die Hebelasche 1 beispielsweise einstückig aus einem Stück Blech beispielsweise aus Aluminium, Edelstahl, einer Metalllegierung oder aber auch aus Kunststoff gefertigt sein, so dass vorteilhafterweise die erste Achse und/oder der Schiebevorsprung 4 einstückig an der Hebelasche 1 ausgebildet ist. Da sich durch Stanzen und Biegen von Blechen auf einfache und effektive Weise dreidimensionale Formen herstellen lassen, ist die Verarbeitung von Blech hierfür besonders bevorzugt und gewährleistet zudem eine dauerhafte Funktionalität.

Um eine möglichst kompakte Gestaltung des Verschlussmechanismusses zu erreichen, zeigt das Anschlagselement 2a des Schiebers 2 eine lochförmige Aussparung, in der der Gegenschieber 3 aufgenommen und geführt wird. Damit entsteht eine wechselseitige Führung des Schiebers und des Gegenschiebers, wodurch eine sichere Translationsbewegung unterstützt ist.

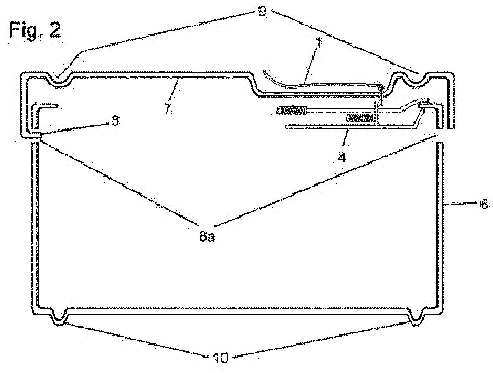

Fig. 2 zeigt eine beispielhafte Ausführungsform eines Siebkorbes gemäß der vorliegenden Erfindung. Ein Siebkorb gemäß der vorliegenden Erfindung kann beispielsweise bei der Aufnahme von medizinischen Instrumenten für die Sterilisation in einem Sterilisierbehälter verwendet werden. Der erfindungsgemäße Siebkorb umfasst einen Behälter 6 zur Aufnahme des zu sterilisierenden Gutes und einen Deckel 7 zum Verschließen des Behälters 6. Sowohl der Behälter 6 wie der Deckel 7 kann aus Blechmaterial, beispielsweise Loch- oder Gitterblech hergestellt sein. Das Blechmaterial kann hierbei metallisch insbesondere aus Aluminium, Edelstahl oder einer Metalllegierung oder aber auch aus Kunststoff wie beispielsweise Polyimid, Polyhydroxybutyrat, Polypropylen, Polytetrafluorethylen, Polyurethan oder Polyester beziehungsweise aus einer Kombination davon sein. Der Behälter 6 umfasst einen nach innen gebogenen, oberen Rand 13, Sieböffnungen und mindestens eine Deckelbefestigungsöffnung 8a. Der obere Rand 13 erstreckt sich hierbei von den Seitenwänden des Behälters 6 senkrecht zum Inneren des Behälters hin. Die Sieböffnungen dienen dazu, dass das Sterilisationsmittel, beispielsweise Flüssigkeit oder aber auch Gas durch die Behälterwand an das zu sterilisierende Gut gelangen kann.

Der Deckel 7 des erfindungsgemäßen Siebkorbes ist über mindestens einen Befestigungsvorsprung 8 mit einer der Deckelbefestigungsöffnungen 8a in Eingriff zu bringen und enthält einen Verschlussmechanismus wie oben beschrieben. Der Befestigungsvorsprung 8 des Deckels 7 und die Deckelbefestigungsöffnung 8a des Behälters 6 müssen miteinander in Eingriff zu bringen sein, so dass der Deckel 7 mit dem Behälter 6 verbunden ist. Bevorzugterweise sind der Befestigungsvorsprung 8 des Deckels 7 und die Deckelbefestigungsöffnung 8a des Behälters 6 auf einer zum Verschlussmechanismus des Deckels gegenüber liegenden Seite angeordnet. Hierzu kann die Deckelbefestigungsöffnung 8a des Behälters 6 beispielsweise als Schlitz ausgebildet sein und der entsprechende Befestigungsvorsprung 8 des Deckels als flacher Vorsprung, der in die schlitzartige Deckelbefestigungsöffnung 8a einführbar ist. Beispielsweise kann der Befestigungsvorsprung 8 des Deckels 7 einstückig mit dem Deckel 7 aus Blechmaterial geformt sein. Es sind jedoch auch andere Kombinationen aus Deckelbefestigungsöffnung und Befestigungsvorsprung 8, wie beispielsweise Loch und zylinderförmiger Vorsprung möglich.

Die Erfindung ist jedoch nicht auf diese konkreten Ausführungsbeispiele beschränkt.

Im geschlossenen Zustand des Siebkorbes greift der Schieber 2 des Verschlussmechanismus unter den bevorzugt als nach innen gebogenen, oberen Rand 13 ausgebildeten Verschlusseingriff 13 des Behälters ein und verhindert zusammen mit dem in Eingriff gebrachten Befestigungsvorsprung 8 ein unbeabsichtigtes Öffnen des Deckels 7.

Der Verschlussmechanismus kann hierbei beispielsweise mittels Nieten oder Schrauben oder aber auch Schweißen oder Löten mit dem Deckel 7 fest verbunden werden. Beim Öffnen des Deckels 7 durch Anheben der Hebelasche 1 verschiebt sich der Schieber 2 senkrecht zur ersten Achse und löst dadurch den Eingriff unter dem Verschlusseingriff 13 insbesondere dem oberen Rand 13 des Behälters. Der Gegenschieber 3 wird durch Federkraft des Federelementes 5b entgegen der Verschieberichtung des Schiebers 2 zum als nach innen gebogenen, oberen Rand 13 ausgebildeten Verschlusseingriff 13 des Behälters 6 hin gedrückt und erzeugt dadurch eine nach oben gerichtete Kraft, die den Deckel 7 in Öffnungsrichtung anhebt. Durch das Anheben des Deckels 7 des Siebkorbes durch den federvorgespannten Gegenschieber 3 erhält der Bediener eine eindeutige Rückmeldung, dass der Verschlussmechanismus ausgerastet ist und der Deckel geöffnet ist. Nach dem Öffnen des Verschlussmechanismusses kann der Bediener den Deckel 7 entweder hochklappen, wobei Deckel 7 und Behälter 6 in Verbindung bleiben, oder aber den Befestigungsvorsprung 8 des Deckels 7 außer Eingriff mit der Deckelbefestigungsöffnung 8a des Behälters 6 bringen und somit den Deckel 7 von dem Behälter 6 des erfindungsgemäßen Siebkorbes lösen.

Das Schließen des erfindungsgemäßen Siebkorbes erfolgt in umgekehrter Reihenfolge, in dem der Deckel heruntergeklappt oder auf den Siebkorb gesetzt und hinuntergedrückt wird. Dabei wird der Schieber 2 mit seinem schrägen Abschnitt 2b durch das Entlanggleiten des nach innen gebogenen, oberen Randes 13 des Behälters 6 an dem schrägen Abschnitt in Verschieberichtung entgegen der Federkraft des Federelementes 5a verschoben und das Federelement 5a gespannt. Parallel dazu wird auch der Gegenschieber 3 mit seinem schrägen Abschnitt 3b mit dem Federelement 5b gespannt. Mit Erreichen der Verschlussposition kann sich der Schieber 2 wieder entgegen der Verschieberichtung bewegen, da die Bewegungseinschränkung durch den als nach innen gebogenen, oberen Rand 13 ausgebildeten Verschlusseingriff 13 entfallen ist. Der Schieber 2 wird durch die Federkraft des vorgespannten Federelementes 5a wieder vorgeschoben und greift unter den als nach innen gebogenen, oberen Rand 13 ausgebildeten Verschlusseingriff 13 des Behälters 6 ein. Weiterhin wird der Gegenschieber 3 durch beispielsweise das Federelement 5b nach vorne entgegen der Verschieberichtung gepresst, sodass der als nach innen gebogene, obere Rand 13 ausgebildete Verschlusseingriff 13 von den Enden des Gegenschiebers 3 und des Schiebers 2 umschlossen und festgelegt ist. Dadurch ist der Deckel 7 über den Befestigungsvorsprung 8 und den Schieber 2 fest mit dem Behälter 6 verbunden.

Der erfindungsgemäße Verschlussmechanismus ermöglicht durch die unterstützende Kraft des Gegenschiebers 3 eine einhändige Bedienung, was insbesondere beim Be- und Entladen des erfindungsgemäßen Siebkorbes für den Anwender von Vorteil sein kann.

Gemäß einer besonders vorteilhaften Ausführungsform und wie in Fig. 2 dargestellt, kann die Hebelasche 1 in einer Vertiefung der Oberseite des Deckels 7 versenkt angeordnet sein und die erste Achse in seitlichen Aussparungen der Vertiefung gelagert werden. Insbesondere bei der Herstellung des Siebkorbes aus Blech ist es auf diese Art und Weise besonders einfach, ein Lager für die Hebelasche 1 ohne separate Bauteile zu bilden.

Gemäß einer besonders bevorzugten Ausführungsform weist der Deckel 7 zwei Befestigungsvorsprünge 8 auf der gleichen Seite auf und der Behälter 6 weist mindestens vier Deckelbefestigungsöffnungen 8a auf, wobei sich immer zwei Deckelbefestigungsöffnungen 8a paarweise gegenüberliegen. Durch diese Anordnung von Deckelbefestigungsöffnungen 8a im Behälter 6 und der zwei Befestigungsvorsprünge 8 an dem Deckel 7 kann der Deckel 7 aufgrund der Symmetrie der Deckelbefestigungsöffnungen 8a in dem Behälter 6 in zwei verschiedenen Richtungen (um 180° gedreht) auf dem Behälter 6 angeordnet werden. Durch das von oben betrachtet sich bildende Dreieck aus den zwei Befestigungsvorsprüngen 8 auf der gleichen Seite des Deckels 7 und dem Verschlussmechanismus auf der gegenüberliegenden Seite des Deckels 7 lässt sich eine besonders sichere Verbindung aus Deckel 7 und Behälter 6 herstellen. Die Erfindung ist jedoch nicht auf diese Ausführungsform beschränkt. Auch beispielsweise ein einzelner breiter Befestigungsvorsprung 8 auf einer Deckelseite gegenüber des Verschlussmechanismusses kann eine sichere Verbindung von Deckel 7 und Behälter 6 herstellen.

Um beispielsweise im Krankenhausbetrieb mehrere Siebkörbe übereinander stapeln zu können, weist ein quaderförmiger Siebkorb gemäß einer besonderen Ausführungsform je eine Stapelmulde 9 in jeder der vier Ecken des Deckels 7 auf und je ein Stapelfuß 10 ist in jeder der vier Ecken des Bodens des Behälters 6 ausgebildet. Dabei ist der Verschlussmechanismus von zwei Stapelmulden 9 im Deckel 7 umfasst, wodurch eine Versteifung des Deckels 7 im Bereich des Verschlussmechanismusses geschaffen ist. Diese Versteifung gewährleistet einen sicheren Betrieb des Siebkorbes auch unter schwierigen Bedingungen. Beim Stapeln mehrerer dieser quaderförmigen Siebkörbe übereinander können so die Stapelfüße eines Siebkorbes mit den Stapelmulden des darunterliegenden Siebkorbes in Eingriff gelangen, um somit beispielsweise beim Transport ein Verrutschen der gestapelten Siebkörbe gegeneinander zu vermeiden.

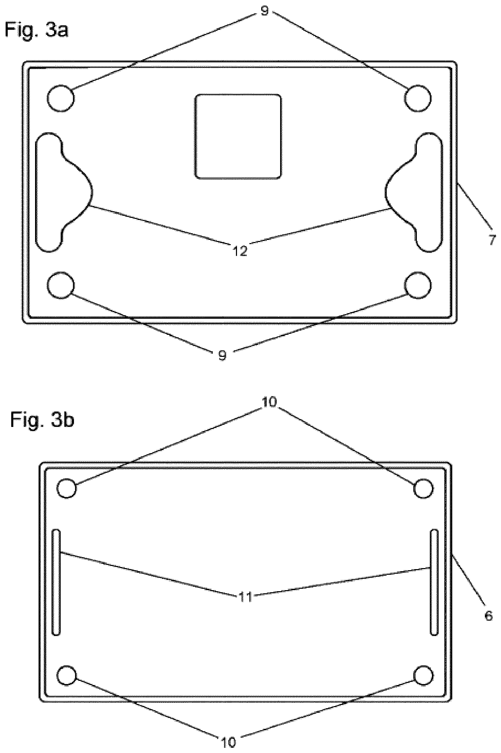

Die Figuren 3a und 3b zeigen eine bevorzugte Ausführungsform eines erfindungsgemäßen Siebkorbes, bei dem auf der Innenseite des Behälters 6 auf zwei gegenüberliegenden Seiten jeweils ein Griffbügel 11 angebracht ist und bei dem im Deckel 7 auf zwei entsprechend gegenüberliegenden Seiten zwei Griffaussparungen 12 angeordnet sind, so dass bei geschlossenem Deckel 7 die Griffbügel 11 des Behälters 6 durch die Griffaussparungen 12 des Deckels 7 durchführbar sind. Hierdurch lässt sich einerseits der Siebkorb an den Griffbügeln 11 sicher transportieren und andererseits bleiben jedoch die Außenseiten des Siebkorbes plan, so dass - beispielsweise bei der Lagerung - selbst bei aneinandergrenzenden Siebkörben sich diese nicht gegeneinander verhaken können. Als vorteilhaftes Material sowohl für den Verschlussmechanismus wie auch für den erfindungsgemäßen Siebkorb können beispielsweise Bleche aus Stahl oder einer stahlumfassenden Legierung, sowie Titan, Aluminium oder Magnesium bzw. deren Legierungen oder aber auch Kunststoff verwendet werden.

Der erfindungsgemäße Siebkorb kann in verschiedensten Geometrien hergestellt werden. Besonders bevorzugt ist jedoch die Quaderform, da diese sehr platzsparend gelagert und transportiert werden kann. Die Siebkörbe können hierbei quadratische oder rechteckige Grundflächen mit unterschiedlichsten Kantenlängen aufweisen. Durch eine Quaderform lassen sich so auch ganze Sets von Siebkörben herstellen, die geometrisch voteilhaft kombiniert werden können, so dass beispielsweise zwei quadratische Siebkörbe auf einen entsprechend rechteckigen passgenau gestapelt werden können. Quaderform bedeutet in diesem Zusammenhang nicht notwendigerweise, dass die Ecken des Quaders scharfkantige 90°-Ecken sein müssen, vielmehr können die Ecken auch abgerundet oder abgeschrägt ausgebildet sein.

### Bezugszeichenliste

- 1: Hebelasche
- 2: Schieber
- 2a: Anschlagselement
- 2b: schräg verlaufender Abschnitt am Schieber 2
- 3: Gegenschieber
- 3a: gerader Endabschnitt
- 3b: schräg verlaufender Abschnitt am Gegenschieber 3
- 4: Schiebevorsprung
- 5a: Federelement
- 5b: Federelement
- 6: Behälter
- 7: Deckel
- 8: Befestigungsvorsprung
- 8a: Deckelbefestigungsöffnung
- 9: Stapelmulde
- 10: Stapelfuß
- 11: Griffbügel
- 12: Griffaussparung
- 13: Verschlusseingriff; nach innen gebogener Rand

## Patentansprüche

1. Verschlussmechanismus für einen Siebkorb mit Deckel mit einem Griff zur Betätigung eines Schiebers zum rastenden Verschluss,
**dadurch gekennzeichnet, dass** der Griff als Hebelasche (1) ausgebildet ist,
und dass der Verschlussmechanismus einen federnd gelagerten Gegenschieber (3) aufweist, wobei die Hebelasche (1) kippbar um eine erste Achse gelagert ist und mit einem Schiebevorsprung (4) verbunden ist, der so ausgebildet ist, dass er für einen Öffnungsvorgang beim Anheben der Hebelasche (1) den Schieber (2) senkrecht zur ersten Achse verschiebt und wobei der Gegenschieber (3) durch Federkraft entgegen der Verschieberichtung des Schiebers (2) gedrückt wird.

2. Verschlussmechanismus nach Anspruch 1, wobei die erste Achse einstückig durch Vorsprünge an der Hebelasche (1) ausgebildet ist.

3. Verschlussmechanismus nach einem der Ansprüche 1 oder 2, wobei der Schiebevorsprung (4) einstückig an der Hebelasche (1) ausgebildet ist.

4. Verschlussmechanismus nach einem der Ansprüche 1 bis 3, wobei der Schieber (2) oder das Anschlagselement (2a) des Schiebers (2) eine Aussparung insbesondere eine lochförmige Aussparung zur Aufnahme des Gegenschiebers (3) und zur wechselseitigen Führung aufweist.

5. Verschlussmechanismus nach einem der Ansprüche 1 bis 4, weiterhin aufweisend wenigstens ein Federelement (5b), das mit dem Gegenschieber (3) zusammenwirkt und geeignet ist, eine Federkraft entgegen der Verschieberichtung des Schiebers (2) zu erzeugen
und/oder weiterhin aufweisend wenigstens ein Federelement (5a), das mit dem Schieber (2) zusammenwirkt und geeignet ist, eine Federkraft entgegen der Verschieberichtung des Schiebers (2) zu erzeugen.

6. Verschlussmechanismus nach einem der Ansprüche 1 bis 5, wobei der Schieber (2) oder Gegenschieber (3) flächig ausgebildet ist und ein Gehäuse bildet, das den restlichen Verschlussmechanismus zumindest teilweise umschließt.

7. Verschlussmechanismus nach einem der Ansprüche 1 bis 6, weiterhin aufweisend einen schräg zur Verschieberichtung verlaufenden Abschnitt (2b, 3b) am Schieber (2) und/oder am Gegenschieber (3).

8. Verschlussmechanismus nach einem der Ansprüche 1 bis 7, weiterhin aufweisend einen Schiebevorsprung (4), der ein Anschlagselement (2a) am Schieber (2) in Verschieberichtung umfasst und/oder der mit dem Schieber (2) mechanisch so verbunden ist, dass eine bidirektionale Bewegung des Schiebevorsprungs (4) in eine bidirektionale Bewegung des Schiebers (2) übertragen wird.

9. Verschlussmechanismus nach einem der Ansprüche 1 bis 8, weiterhin aufweisend wenigstens einen zusätzlichen Schieber (2) und wenigstens einen zusätzlichen Gegenschieber (3), die gemeinsam einer Hebelasche (1) mit Schiebevorsprung (4) so zugeordnet sind, dass der Schiebevorsprung (4) für einen Öffnungsvorgang beim Anheben der Hebelasche (1) die Schieber (2) senkrecht zur ersten Achse verschiebt und dass die Gegenschieber (3) durch Federkraft entgegen der Verschieberichtung des Schiebers (2) gedrückt werden.

10. Siebkorb umfassend
einen mit mindestens einem Verschlusseingriff (13) und Sieböffnungen versehenen Behälter (6); einen Deckel (7), der wenigstens einen Verschlussmechanismus nach einem der Ansprüche 1 bis 9 aufweist;
wobei im geschlossenen Zustand des Siebkorbes der Schieber (2) des oder der Verschlussmechanismen unter den oder die Verschlusseingriffe (13) des Behälters (6) eingreifen und ein unbeabsichtigtes Öffnen des Deckels (7) verhindern und
wobei beim Öffnen des Deckels (7) durch Anheben der Hebelasche (1) der Verschlussmechanismen sich der Schieber (2) senkrecht zur ersten Achse verschiebt und dadurch den Eingriff unter den oder die Verschlusseingriffe (13) des Behälters (6) löst und den Deckel (7) zum Öffnen frei gibt.

11. Siebkorb umfassend
einen mit mindestens einem Verschlusseingriff (13), Sieböffnungen und mindestens einer Deckelbefestigungsöffnung (8a) versehenen Behälter (6); einen Deckel (7), der über mindestens einen Befestigungsvorsprung (8) mit einer der Deckelbefestigungsöffnungen (8a) in Eingriff bringbar ist und der einen Verschlussmechanismus nach einem der Ansprüche 1 bis 9 aufweist;
wobei im geschlossenen Zustand des Siebkorbes der Schieber (2) des Verschlussmechanismusses unter den oder die Verschlusseingriffe (13) des Behälters (6) eingreift und zusammen mit dem in Eingriff gebrachten Befestigungsvorsprung (8) ein unbeabsichtigtes Öffnen des Deckels (7) verhindert und
wobei beim Öffnen des Deckels (7) durch Anheben der Hebelasche (1) sich der Schieber (2) senkrecht zur ersten Achse verschiebt und dadurch den Eingriff unter den oder die Verschlusseingriffe (13) des Behälters (6) löst und den Deckel (7) zum Öffnen frei gibt.

12. Siebkorb nach Anspruch 10 oder 11, wobei beim Öffnen des Deckels (7) durch Anheben der Hebelasche (1) der Verschlussmechanismen sich der Schieber (2) senkrecht zur ersten Achse verschiebt und dadurch den Eingriff unter den oder die Verschlusseingriffe (13) des Behälters (6) löst und der Gegenschieber (3) durch Federkraft entgegen der Verschieberichtung des Schiebers (2) zum Verschlusseingriff (13) des Behälters (6) hin gedrückt wird und eine nach oben gerichtete Kraft erzeugt.

13. Siebkorb nach einem der Ansprüche 10 bis 12, wobei mehrere Verschlüsse nach einem der Ansprüche 1 bis 9 am Deckel vorgesehen sind, die an verschiedenen Seiten des Deckels (7) insbesondere an gegenüberliegenden Seiten des Deckels (7) verteilt angeordnet sind.

14. Siebkorb nach einem der Ansprüche 10 bis 13, wobei die Hebelasche (1) in einer Vertiefung der Oberseite des Deckels (7) versenkt angeordnet ist und die erste Achse in seitlichen Aussparungen der Vertiefung gelagert ist.

15. Siebkorb nach einem der Ansprüche 10 bis 14, wobei der Deckel (7) mindestens zwei Befestigungsvorsprünge (8) auf der gleichen Seite und gegenüber des Verschlussmechanismusses aufweist und der Behälter (6) mindestens zwei insbesondere vier Deckelbefestigungsöffnungen (8a) aufweist und wobei sich immer zwei Deckelbefestigungsöffnungen (8a) paarweise gegenüberliegen.

## Claims

1. Closure mechanism for a perforated basket with a lid, having a handle for actuating a slide for latching closure, **characterized in that** the handle is designed as a lifting tab (1), and **in that** the closure mechanism has a spring-mounted counter-slide (3), wherein the lifting tab (1) is mounted such that it can be tilted about a first axis and is connected to a sliding projection (4) which is designed in such a way that, for an opening operation when lifting the lifting tab (1), it displaces the slide (2) perpendicularly to the first axis, and wherein the counter-slide (3) is pressed by a spring force counter to the displacement direction of the slide (2).

2. Closure mechanism according to Claim 1, wherein the first axis is formed in one piece by way of projections on the lifting tab (1).

3. Closure mechanism according to either of Claims 1 and 2, wherein the sliding projection (4) is formed in one piece on the lifting tab (1).

4. Closure mechanism according to one of Claims 1 to 3, wherein the slide (2) or the stop element (2a) of the slide (2) has a cutout, in particular a hole-shaped cutout, for receiving the counter-slide (3) and for mutual guidance.

5. Closure mechanism according to one of Claims 1 to 4, further having at least one spring element (5b) which interacts with the counter-slide (3) and is suitable for generating a spring force counter to the displacement direction of the slide (2), and/or further having at least one spring element (5a) which interacts with the slide (2) and is suitable for generating a spring force counter to the displacement direction of the slide (2).

6. Closure mechanism according to one of Claims 1 to 5, wherein the slide (2) or counter-slide (3) is of flat design and forms a housing which at least partially encloses the remainder of the closure mechanism.

7. Closure mechanism according to one of Claims 1 to 6, further having a portion (2b, 3b) on the slide (2) and/or on the counter-slide (3) that extends obliquely with respect to the displacement direction.

8. Closure mechanism according to one of Claims 1 to 7, further having a sliding projection (4) which comprises a stop element (2a) on the slide (2) in the displacement direction and/or which is mechanically connected to the slide (2) in such a way that a bidirectional movement of the sliding projection (4) is converted into a bidirectional movement of the slide (2).

9. Closure mechanism according to one of Claims 1 to 8, further having at least one additional slide (2) and at least one additional counter-slide (3) which are jointly assigned a lifting tab (1) with a sliding projection (4) in such a way that, for an opening operation when lifting the lifting tab (1), the sliding projection (4) displaces the slide (2) perpendicularly to the first axis and in such a way that the counter-slides (3) are pressed by spring force counter to the displacement direction of the slide (2).

10. Perforated basket comprising
a container (6) which is provided with at least one closure engagement portion (13) and openings;
and a lid (7) which has at least one closure mechanism according to one of Claims 1 to 9; wherein, in the closed state of the perforated basket, the slide (2) of the closure mechanism or mechanisms engage below the closure engagement portion or portions (13) of the container (6) and prevent an unintentional opening of the lid (7), and wherein, during opening of the lid (7) by lifting the lifting tab (1) of the closure mechanisms, the slide (2) is displaced perpendicularly to the first axis and thereby releases the engagement below the closure engagement portion or portions (13) of the container (6) and frees the lid (7) for opening.

11. Perforated basket comprising
a container (6) which is provided with at least one closure engagement portion (13), openings and at least one lid fastening opening (8a);
and a lid (7) which can be brought into engagement with one of the lid fastening openings (8a) via at least one fastening projection (8) and which has a closure mechanism according to one of Claims 1 to 9;
wherein, in the closed state of the perforated basket, the slide (2) of the closure mechanism engages below the closure engagement portion or portions (13) of the container (6) and, together with the fastening projection (8) brought into engagement, prevents an unintentional opening of the lid (7), and
wherein, during opening of the lid (7) by lifting the lifting tab (1), the slide (2) is displaced perpendicularly to the first axis and thereby releases the engagement below the closure engagement portion or portions (13) of the container (6) and frees the lid (7) for opening.

12. Perforated basket according to Claim 10 or 11, wherein, during opening of the lid (7) by lifting the lifting tab (1) of the closure mechanisms, the slide (2) is displaced perpendicularly to the first axis and thereby releases the engagement below the closure engagement portion or portions (13) of the container (6), and the counter-slide (3) is pressed towards the closure engagement portion (13) of the container (6) by spring force counter to the displacement direction of the slide (2) and generates an upwardly directed force.

13. Perforated basket according to one of Claims 10 to 12, wherein a plurality of closures according to one of Claims 1 to 9 are provided on the lid and are arranged in a distributed manner on different sides of the lid (7), in particular on opposite sides of the lid (7).

14. Perforated basket according to one of Claims 10 to 13, wherein the lifting tab (1) is arranged sunk in a depression of the upper side of the lid (7) and the first axis is mounted in lateral cutouts of the depression.

15. Perforated basket according to one of Claims 10 to 14, wherein the lid (7) has at least two fastening projections (8) on the same side and opposite the closure mechanism, and the container (6) has at least two, in particular four, lid fastening openings (8a), and wherein always two lid fastening openings (8a) are situated opposite one another in pairs.

## Revendications

1. Mécanisme de verrouillage pour une crépine dotée d'un couvercle avec une poignée pour l'actionnement d'un coulisseau pour le verrouillage maintenu, **caractérisé en ce que** la poignée est réalisée sous la forme d'une patte de levage (1), et **en ce que** le mécanisme de verrouillage présente un coulisseau opposé (3) monté à ressort, dans lequel la patte de levage (1) est montée de façon pivotante autour d'un premier axe et est reliée à une saillie de coulisseau (4), qui est réalisée de telle manière que pour une opération d'ouverture lors du levage de la patte de levage (1) elle déplace le coulisseau (2) perpendiculairement au premier axe et dans lequel le coulisseau opposé (3) est poussé par la force du ressort à l'inverse de la direction de déplacement du coulisseau (2) .

2. Mécanisme de verrouillage selon la revendication 1, dans lequel le premier axe est réalisé d'une seule pièce à travers des saillies sur la patte de levage (1).

3. Mécanisme de verrouillage selon une des revendications 1 ou 2, dans lequel la saillie de coulisseau (4) est réalisée d'une seule pièce sur la patte de levage (1).

4. Mécanisme de verrouillage selon l'une quelconque des revendications 1 à 3, dans lequel le coulisseau (2) ou l'élément de butée (2a) du coulisseau (2) présente une découpe, en particulier une découpe en forme de trou, destinée à contenir le coulisseau opposé (3) et pour le guidage mutuel.

5. Mécanisme de verrouillage selon l'une quelconque des revendications 1 à 4, présentant en outre au moins un élément de ressort (5b), qui coopère avec le coulisseau opposé (3) et qui convient pour produire une force de ressort à l'inverse de la direction de déplacement du coulisseau (2), et/ou présentant en outre au moins un élément de ressort (5a), qui coopère avec le coulisseau (2) et qui convient pour produire une force de ressort à l'inverse de la direction de déplacement du coulisseau (2) .

6. Mécanisme de verrouillage selon l'une quelconque des revendications 1 à 5, dans lequel le coulisseau (2) ou le coulisseau opposé (3) est de forme plate et forme un boîtier, qui entoure au moins en partie le reste du mécanisme de verrouillage.

7. Mécanisme de verrouillage selon l'une quelconque des revendications 1 à 6, présentant en outre sur le coulisseau (2) et/ou sur le coulisseau opposé (3) une partie (2b, 3b) s'étendant en oblique par rapport à la direction de déplacement.

8. Mécanisme de verrouillage selon l'une quelconque des revendications 1 à 7, présentant en outre une saillie de coulisseau (4), qui comprend un élément de butée (2a) sur le coulisseau (2) dans la direction de déplacement et/ou qui est mécaniquement lié au coulisseau (2) de telle manière qu'un mouvement bidirectionnel de la saillie de coulisseau (4) soit transmis dans un mouvement bidirectionnel du coulisseau (2).

9. Mécanisme de verrouillage selon l'une quelconque des revendications 1 à 8, présentant en outre au moins un coulisseau supplémentaire (2) et au moins un coulisseau opposé supplémentaire (3), qui sont associés ensemble à une patte de levage (1) avec une saillie de coulisseau (4), de telle manière que la saillie de coulisseau (4) déplace pour une opération d'ouverture lors du levage de la patte de levage (1) le coulisseau (2) perpendiculairement au premier axe et que les coulisseaux opposés (3) soient poussés par la force de ressort à l'inverse de la direction de déplacement du coulisseau (2) .

10. Crépine comprenant
un réservoir (6) doté d'au moins un arrêt de verrouillage (13) et d'ouvertures de crépine;
un couvercle (7), qui présente au moins un mécanisme de verrouillage selon l'une quelconque des revendications 1 à 9;
dans laquelle, dans l'état fermé de la crépine, le coulisseau (2) du ou des mécanisme(s) de verrouillage s'engagent en dessous du ou des arrêt(s) de verrouillage (13) du réservoir (6) et empêchent une ouverture inopinée du couvercle (7), et
dans laquelle, lors de l'ouverture du couvercle (7) par levage de la patte de levage (1) des mécanismes de verrouillage, le coulisseau (2) se déplace perpendiculairement au premier axe et rompt ainsi l'engagement en dessous du ou des arrêt(s) de verrouillage (13) du réservoir (6) et libère le couvercle (7) pour l'ouverture.

11. Crépine comprenant
un réservoir (6) doté d'au moins un arrêt de verrouillage (13), d'ouvertures de crépine et d'au moins une ouverture de fixation de couvercle (8a);
un couvercle (7), qui peut être mis en prise avec une des ouvertures de fixation de couvercle (8a) au moyen d'au moins une saillie de fixation (8) et qui présente un mécanisme de verrouillage selon l'une quelconque des revendications 1 à 9;
dans laquelle, dans l'état fermé de la crépine, le coulisseau (2) du mécanisme de verrouillage s'engage en dessous du ou des arrêt (s) de verrouillage (13) du réservoir (6) et, de concert avec la saillie de fixation (8) mise en prise, empêche une ouverture inopinée du couvercle (7), et
dans laquelle, lors de l'ouverture du couvercle (7) par levage de la patte de levage (1), le coulisseau (2) se déplace perpendiculairement au premier axe et rompt ainsi l'engagement en dessous du ou des arrêt(s) de verrouillage (13) du réservoir (3) et libère le couvercle (7) pour l'ouverture.

12. Crépine selon la revendication 10 ou 11, dans laquelle, lors de l'ouverture du couvercle (7) par levage de la patte de levage (1) des mécanismes de verrouillage, le coulisseau (2) se déplace perpendiculairement au premier axe et rompt de ce fait l'engagement en dessous du ou des arrêt (s) de verrouillage (13) du réservoir (6) et le coulisseau opposé (3) est poussé par la force de ressort à l'inverse de la direction de déplacement du coulisseau (2) vers l'arrêt de verrouillage (13) du réservoir (6) et produit une force dirigée vers le haut.

13. Crépine selon l'une quelconque des revendications 10 à 12, dans laquelle il est prévu sur le couvercle plusieurs verrous selon l'une quelconque des revendications 1 à 9, qui sont disposés sur différents côtés du couvercle (7), en particulier répartis sur des côtés opposés du couvercle (7).

14. Crépine selon l'une quelconque des revendications 10 à 13, dans laquelle la patte de levage (1) est disposée de façon abaissée dans un creux du côté supérieur du couvercle (7) et le premier axe est supporté dans des découpes latérales du creux.

15. Crépine selon l'une quelconque des revendications 10 à 14, dans laquelle le couvercle (7) présente au moins deux saillies de fixation (8) sur le même côté et en face du mécanisme de verrouillage et le réservoir (6) présente au moins deux, en particulier quatre, ouvertures de fixation de couvercle (8a) et dans laquelle deux ouvertures de fixation de couvercle (8a) se font toujours face mutuellement par paires.
